# EUROPEAN PATENT APPLICATION

(11) **EP 4 360 539 A1**
(43) Date of publication of application: **01.05.2024**
(21) Application number: 23178238.4
(22) Date of filing: 08.06.2023
(51) Int. Cl.: A61B 5/00, B41J 2/21

(54) **TASTE TESTER, TEST KIT COMPRISING THE TESTER, METHOD FOR EVALUATING DATA COLLECTION FROM THE TEST KIT APPLICATION, AND SYSTEM FOR DATA COLLECTION**

(30) Priority: 09.06.2022 CZ 20220025; 09.06.2022 CZ 202239991 U
(71) Applicant: Univerzita Pardubice, 530 09 Pardubice, Polabiny (CZ)
(72) Inventor: Syrovy, Tomas, 56501 Sudlickova Lhota (CZ); Vodicka, Jan, 53301 Pardubice (CZ); Dolezal, Petr, 53002 Pardubice (CZ)
(74) Representative: Sedlák, Jirí

(57) **Abstract**

The taste tester (1) comprises the carrier substrate (2), on which at least one test region (3, 3') is formed by the print layer (4) made of a material comprising the taste stimulants for detecting sweet, salty, bitter, sour, and umami taste. Further, the tester is provided with the optical identification member (5) comprising the handling symbol (16) to instruct in the proper use of the tester (1). Further, at least two testers (1) according to the present invention are included in the test kit (7), wherein the data from the application is collected by the system comprising evaluation software module (9) which is linked to the evaluation device (8). The evaluation device (8) is equipped with the camera/camcorder (12) and linked to the database (14) containing information on testers (1). The camera/camcorder (12) is used to take picture/image of the optical identification member (5) of the tester (1) and the evaluation software module (9) of the evaluation device (8) subsequently identifies the tester (1) type by comparison with the data in the database (14).

## Description

### Field of the Invention

The invention relates to the field of healthcare, in particular to the field of taste testing of a test subject, more particularly to a taste tester, a test kit comprising this tester, a method of collecting data from the test kit application, and a system for collecting the data.

### Background of the Invention

The assessment of the taste senses of a test subject is used mainly to determine the symptoms of various diseases, the after-effects of recovery from illness or to detect partial or complete loss of taste. Testers or test kits for assessing the sense of taste are most often based on the principle of stimulating taste receptors in the form of buds located in the tongue papillae and, thus, generally consist of filter paper impregnated with a flavoring agent or taste stimulant. Such an arrangement allows the examination of individual taste sensations such as salty, sweet, bitter, sour, and possibly umami. The disadvantage is that this arrangement is separate for each tongue side, and furthermore, the presence of an investigator is required. Another option is the application of flavorings in the form of drops or candy. These examinations do not allow for lateral examination.

A number of basic tastes are used to test the sense of taste, namely sweet, bitter, sour, and salty. For a number of substances that stimulate taste receptors, there are generally set concentrations of taste stimulants that a test subject with a healthy sense of taste should recognize. For the salty taste, NaCl solution of concentration of 0.016 to 0.25 g/ml is commonly used. For the sweet taste, sucrose solution of concentration of 0.1 to 0.5 g/ml is used. For the sour taste, citric acid solution of concentration of 0.05 to 0.3 g/ml is used. For the bitter taste, a solution of quinine hydrochloride of concentration of 0.0004 to 0.006 g/ml is used. Possible compositions of taste stimulants are also described in KR 20100128576. However, there is still a disadvantage consisting in that the fifth basic taste, namely umami, is not examined in routine practice, as well as the possibility of storing records on remote storage and the need for the presence of a trained person or doctor.

The term "taste stimulant" for the purpose of describing the present invention is defined to mean that the flavoring agent itself causes activation of the taste receptors or taste buds on the tongue surface.

WO 2020/070256 describes an apparatus for testing taste quality and sensitivity comprising an electromechanical cartridge for automated preparation of taste stimulants, control terminals, and cloud data storage. The disadvantages of such an apparatus consist in that it requires maintenance, it is expensive and it cannot be used for self-examination of a tested person outside a specialized facility.

This disadvantage is partially eliminated by the taste testing apparatus described in CN 211583101. The said document describes a set of taste stimulant capsules that can be used for taste testing easily and anywhere; however, there is still the disadvantage consisting in that such a device cannot be used for unilateral/bilateral taste testing.

Therefore, the invention object is to provide such a taste tester and a test kit comprising such a tester, which would allow for self-examination of the taste sensations of a test subject, wherein such a tester and/or test kit would include a number of basic tastes including umami, allowing for unilateral and bilateral taste testing. A further invention object is to provide such a taste tester and a test kit comprising such a tester, which would be prepared using printing or coating techniques to reduce the production costs. Another invention object is a method for evaluating the data collection from a test kit application and a system for collecting such data so that the output of the taste sensation testing process could be stored on an external storage device that would be linked to a physician's office.

### Summary of the invention

The task set forth is solved by using a taste tester according to the present invention. Summary of the invention consists in that the tester comprises a carrier substrate on which at least one test region is formed by a print layer of a material comprising a taste stimulant. The tester prepared in this way is suitable for self-examination of a tested subject outside the physician's office and the cost of preparation of such a tester is low in relation to the preparation by the printing method. The amount of taste stimulants can be controlled or defined by the printed thickness of the print layer and the area thereof.

The term "taste stimulant print layer" means the print layer composition that includes a selected taste stimulant from the group for detecting sweet, salty, bitter, sour, or umami tastes, said print layer further comprising a water-soluble polymer, a volatile surfactant, a humectant, a monofunctional alcohol, and water.

In the preferred embodiment, the print layer material comprises taste stimulant for detecting sweet taste selected from the group of: sucrose, glucose, fructose, sorbitol, aspartame, sodium cyclamate, saccharin, potassium acesulfame, sucralose, thaumatin, neotame, lugduname, and/or combinations thereof. The taste stimulants chosen in this way to detect the sweet taste are inexpensive, readily available, and even lower amounts are sufficient to detect taste receptor cell activity on the tongue surface and, thus, layers of lower concentrations can be printed.

In another preferred embodiment, the print layer material comprises taste stimulant for detecting salty taste selected from the group of: NaCl, KCI, KBr, NaBr, LiCI, LiBr, Lil, NaNO₃, KNO₃, L-ornithyl-β-alanine, and/or combinations thereof. The taste stimulants chosen in this way to detect the salty taste are well known to humans and are easily detected by taste receptor cells on the tongue surface even at low concentrations.

In another preferred embodiment, the print layer material comprises taste stimulant for detecting bitter taste selected from the group of: quinine, 6-n-propylthiouracile, phenylthiourea, sucrose octacetate, caffeine, strychnine, naringin, neohesperidine, humulone, and/or combinations thereof. The bitter taste stimulants prepared in this way is easily detectable by the taste buds.

In another preferred embodiment, the print layer material comprises taste stimulant for detecting sour taste selected from the group of: citric acid, acetic acid, ethylenediaminetetraacetic acid, tartaric acid, phosphoric acid, ascorbic acid, and/or combinations thereof. When choosing the sour taste stimulant, toxicity must also be taken into account, given that acids are involved many times. The selected taste stimulants for detecting sour taste according to the present invention are acids frequently used in gastronomy, which are known for their relatively low toxicity.

In another preferred embodiment, the print layer material comprises taste stimulant for detecting umami taste selected from the group of: monosodium glutamate, potassium glutamate, and/or combinations thereof. The umami taste is defined by these substances, which are often found in Asian cuisine, from where this taste is known.

In the preferred embodiment, two test regions are arranged parallel to each other on the carrier substrate at a distance of 1 to 30 mm for bilateral taste testing. Such an arrangement is preferrable for the preparation of a tester suitable for the tongue bilateral examination, whereby the individual test regions do not interfere with each other and, thus, the results for the individual tongue parts are not affected by the other one of the tester's group of test regions.

In the further preferred embodiment, there are two test regions on the carrier substrate of which the first test region and the second test region are formed by a print layer of a material comprising the same taste stimulants. This arrangement allows the tongue to be examined bilaterally to the same taste and, therefore, the result is accentuated by being detected on both sides of the tongue.

In another preferred embodiment, there are two test regions on the carrier substrate, wherein the first test region is formed by a print layer of material comprising test stimulants, and the second test region is formed by a print layer of material comprising test stimulants different from the taste stimulants of the first test region. Such an arrangement is preferable for detecting the quality of taste bud response to more complex tastes.

In another preferred embodiment, there are two test regions on the carrier substrate, wherein the first test region is formed by a print layer of material comprising test stimulants, and the second test region is formed by a print layer of material not comprising test stimulants. Such an arrangement is preferable for detecting taste bud activity especially on one side of the tongue.

In the preferred embodiment, the tester further comprises an optical identification device arranged on the carrier substrate identifiable visually or by a camera/camcorder, wherein the optical identification member is preferably a colored array. This arrangement ensures simple and easy handling the tester, especially in the case of self-examination.

In further preferred embodiment, the optical identification member is provided with a handling symbol indicating the tester handling direction. This arrangement is preferable for easy handling the tester and ensures safe handling when the tester is correctly positioned on the tongue surface.

In the preferred embodiment, the tester further comprises a protective layer in the form of an overlaying water-soluble film arranged on the carrier substrate wherein it overlays the test region. This protective layer is mainly used to preserve the test region characteristics for a longer period of time, thus extending the tester lifetime.

In further preferred embodiment, the tester further comprises a protective layer in the form of a removable overlaying film arranged on the carrier substrate, wherein it overlays the test region. Such an arrangement is preferable for direct and rapid application of the tester after removal of the protective layer, wherein the protective layer removal does not alter the properties of the test region.

In the preferred embodiment, the carrier substrate comprises a polymeric film selected from the group of: polyethylene terephthalate, polyvinyl chloride, polyethylene naphthalate, polycarbonate, polypropylene, polyethylene, polyetheretherketone, polystyrene, and/or combinations thereof. Such an arrangement is preferable especially due to the stable characteristics of the selected polymer films and, therefore, such an arrangement prolongs the tester lifetime. At the same time, the possibility to print the test region on selected polymer films is preferable due to the fact that these test regions can be subsequently printed on 3D surfaces formed by these polymer films.

In another preferred embodiment, the carrier substrate is formed by a biocompatible polymer selected from the group of: polysaccharide, proteins, cellulose and/or derivatives thereof, pectins, and/or combinations thereof. Such an arrangement is preferable due to the possible recycling of the used tester prepared on such a substrate.

The summary of the invention further consists in the test kit comprising at least two taste testers according to the present invention. Such a test kit is preferable for a comprehensive examination of the test subject's taste, especially because it is possible to use this test kit for a comprehensive self-examination.

In the preferred embodiment, the test kit comprises exactly 5 testers, each of which contains a test region formed by a print layer of material containing a different taste stimulant. One tester preferably comprises a test region formed by a print layer coated with a taste stimulant for detecting sweet taste. The second tester preferably comprises a test region formed by a print layer coated with a taste stimulant for detecting salty taste. The third tester preferably comprises a test region formed by a print layer coated with a taste stimulant for detecting bitter taste. The fourth tester preferably comprises a test region formed by a print layer coated with a taste stimulant for detecting sour taste. The fifth tester of the test kit preferably comprises a test region formed a print layer coated with a taste stimulant for detecting umami taste. Such an arrangement is preferable for the examination of taste buds of all tastes, especially in the case of testing the test subject by self-examination.

The summary of the invention further consists in a method of collecting data from the tester and/or the test kit according to the present invention. The data collection method comprises successive procedure steps:
a) The tester is placed into the field of view of a camera/camcorder equipped with or associated with an evaluation device, which includes an evaluation software module linked to a database containing tester information and a communication block for remote communication;
b) The camera/camcorder is activated and a picture/image of the optical identification member of the tester is taken;
c) The picture/image is transferred to the evaluation software module, which identifies the tester type by comparing it with the data in the database;
d) With the test region, the tester is placed on the tongue surface of the subject being tested;
e) The test subject tastes the taste stimulants contained in the print layer material forming the test region;
f) The result of the tasting is recorded in the evaluation software module as information about a positive or negative reaction to the taste stimulants contained in the print layer of the test region;
g) The data from the evaluation software module is sent from the communication block to an external storage or a remote server or to the Internet.

For the purposes of the present invention description, the term "evaluation device" refers to an electronic device having a display which is a touchscreen in a preferred embodiment. These include mobile phones, tablets, laptops, and desktops.

For the purposes of the present invention description, the term "database" refers to a collection of information about all combinations of testers that are produced and detected by the optical identification member. Such a database is a part of the application contained in the evaluation device and is linked to the evaluation software module.

For the purposes of the present invention description, the term "recording of tasting results" means entering the subjective evaluation of the tester use on the tongue surface into an application contained in the evaluation device, wherein the recording is preferably on a touchscreen evaluation device.

In the preferred embodiment, between procedure steps d) and e), the camera/camcorder is activated and a picture/image of the test subject with the tester applied on the tongue is taken to evaluate the correct tester placement. Such an arrangement is preferable to control that testing by the self-examination technique is free from potential defects that could affect the test results.

In the further preferred embodiment, the type of taste perceived during the tasting is also recorded as a result of the tasting in the step f). This arrangement is preferable for assessing the extent of any damage to the test subject's taste buds.

In the further preferred embodiment, the left or right side of the tongue is also recorded as a result of the tasting in step f). This arrangement ensures that the collected data on individual test subjects is safely stored even in the event of damage to the evaluation device.

In the further preferred embodiment, the particular type of taste perceived by the left or right side of the tongue is also recorded as a result of the tasting in step f). Such evaluation provides the most comprehensive and the most effective taste testing.

The summary of the invention further consists in a system for performing the data collection method according to the present invention. The system includes at least one tester according to the present invention or at least one test kit according to the present invention. The system further comprises a camera/camcorder for capturing a picture/image of the optical identification member of the tester. The system further includes an evaluation device linked to the camera/camcorder, wherein the evaluation device includes an evaluation software module to evaluate a picture/image of the optical identification member of the tester. The evaluation software module is linked to a database containing stored information about testers, and a communication block linked to the evaluation software module for sending data from the evaluation software module. Such an arrangement is preferable for the safe testing of the test subjects and ensures the reliability of the results, in particular due to the possibility of checking with pictures/images.

In the preferred embodiment, the system further includes an external data storage to store data sent via the communication block from the evaluation software module. Such an arrangement ensures secure storage and collection of data from test subjects to an external storage, which can then be linked to the physician's office.

In another preferred embodiment, the system further includes av remote server to store data sent via the communication block from the evaluation software module. In another preferred embodiment, the system further includes means for connecting and transmitting data to the Internet sent via the communication block from the evaluation software module. This arrangement is preferable for storing data outside the evaluation device or physician's office, wherein both the test subject and the physician still have access to the results.

In particular, the advantage of the taste tester and test kit comprising the tester according to the present invention consists in that it allows for self-examination of the test subject's taste sensations, wherein the tester and/or test kit comprises a number of basic tastes including umami, and allows for unilateral and bilateral taste testing. Another advantage of the taste tester and the test kit comprising the taste tester according to the present invention consists in that it is prepared using printing or coating techniques to reduce production costs. Another advantage of the method for evaluating the data collection from the test kit application and the system for collecting such data according to the present invention consists in that the output of the taste sensation testing process can be stored on an external storage device that is linked to a physician's office.

### Explanation of Drawings

The said invention will be explained in more detail in the following illustrations, wherein:

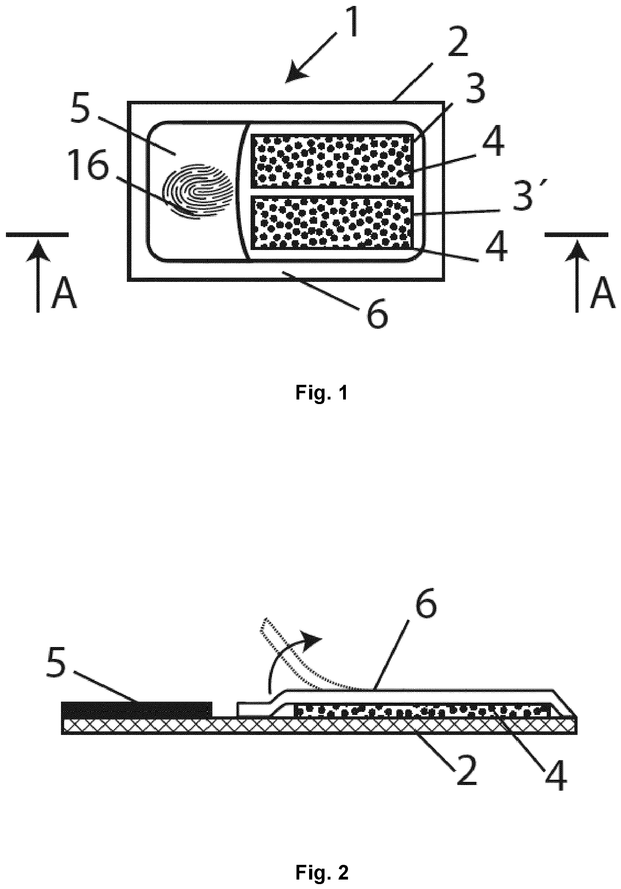

- Fig. 1: shows the top view of the tester;
- Fig. 2: shows a cross-section of the tester;

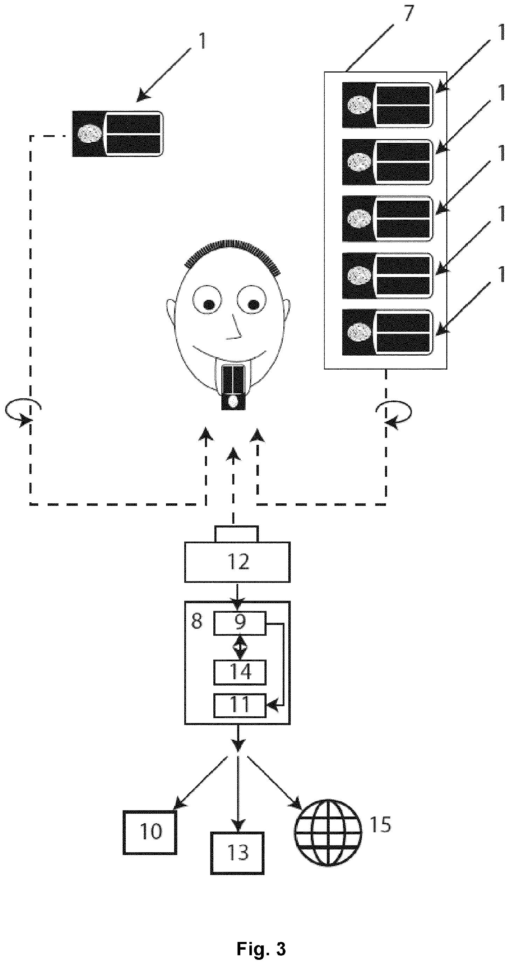

- Fig. 3: shows a block diagram of the system.

### Examples of Invention Embodiments

### Example 1

The tester 1 included the carrier substrate 2 coated with taste stimulants in the test region 3, 3' in the form of the print layer 4. The carrier substrate 2 was made of polyethylene terephthalate. In another example embodiment not shown, the carrier substrate 2 was selected from the group of: polyvinyl chloride, polyethylene naphthalate, polycarbonate, polypropylene, polyethylene, polyetheretherketone, polystyrene, and/or combinations thereof. In another example embodiment not shown, the carrier substrate 2 is formed by a biocompatible polymer selected from the group of: polysaccharide, proteins, cellulose and/or derivatives thereof, pectins, and/or combinations thereof. Two test regions 3, 3', namely the right and left, were printed on the carrier substrate 2. These test regions 3, 3' were printed parallel to each other at a distance of 15 mm. In another example embodiment not shown, these test regions 3, 3' were printed parallel to each other at a distance of 1 to 30 mm. A taste stimulant in the form of fructose to detect the sweet taste was printed on the right test region 3. In another example embodiment not shown, the taste stimulant for detecting sweet taste was selected from the group of: sucrose, glucose, fructose, sorbitol, aspartame, sodium cyclamate, saccharin, potassium acesulfame, sucralose, thaumatin, neotame, lugduname, and/or combinations thereof. A taste stimulant in the form of potassium bromide to detect the salty taste was printed on the left test region 3'. In another example embodiment not shown, the taste stimulant for detecting salty taste was selected from the group of: NaCl, KCI, NaBr, LiCI, LiBr, Lil, NaNO₃, KNO₃, L-ornithyl-β-alanine, and/or combinations thereof. In further example embodiment not shown, a combination was selected from the group of tastes: sweet-bitter, sweet-sour, sweet-umami, salty-bitter, salty-sour, salty-umami, bitter-sour, bitter-umami or sour-umami.

The tester 1 included the carrier substrate 2 coated with taste stimulants in the test region 3, 3' in the form of the print layer 4. The carrier substrate 2 was made of pectin. In other example embodiment not shown, the carrier substrate 2 was selected from the group of: polysaccharide, proteins, cellulose and/or derivatives thereof, and/or combinations thereof. In another example embodiment not shown, the carrier substrate 2 was made of a polymeric film selected from the group of: polyethylene terephthalate, polyethylene naphthalate, polycarbonate, polypropylene, polyethylene, polyetheretherketone, polystyrene, and/or combinations thereof. Two test regions 3, 3', namely the right and left, were printed on the carrier substrate 2. These test regions 3, 3' were printed parallel to each other at a distance of 15 mm. In another example embodiment not shown, these test regions 3, 3' were printed parallel to each other at a distance of 1 to 30 mm. The same taste stimulant in the form of caffeine to detect the bitter taste was printed on both test regions 3, 3'. In another example embodiment not shown, the taste stimulant for detecting bitter taste was selected from the group of: quinine, 6-n-propylthiouracile, phenylthiourea, sucrose octacetate, strychnine, naringin, neohesperidine, humulone, and/or combinations thereof. In another example embodiment not shown, the same taste stimulant selected from the taste group of: sweet, salty, sour and/or umami was printed on both test regions 3, 3'.

The tester 1 included the carrier substrate 2 coated with taste stimulants in the test region 3, 3' in the form of the print layer 4. The carrier substrate 2 was made of polyvinyl chloride polycarbonate. In another example embodiment not shown, the carrier substrate 2 was selected from the group of: polyethylene terephthalate, polyethylene naphthalate, polyvinyl chloride, polypropylene, polyethylene, polyetheretherketone, polystyrene, and/or combinations thereof. In another example embodiment not shown, the carrier substrate 2 is formed by a biocompatible polymer selected from the group of: polysaccharide, proteins, cellulose and/or derivatives thereof, pectins, and/or combinations thereof. Two test regions 3, 3', namely the right and left, were printed on the carrier substrate 2. These test regions 3, 3' were printed parallel to each other at a distance of 15 mm. In another example embodiment not shown, these test regions 3, 3' were printed parallel to each other at a distance of 1 to 30 mm. A taste stimulant in the form of monosodium glutamate to detect the umami taste was printed on the first test region 3. In another example embodiment not shown, the taste stimulant for detecting the umami taste was potassium glutamate or a combination of potassium glutamate and monosodium glutamate. In another example embodiment not shown, the taste stimulant selected from the taste group of: sweet, salty, bitter and/or sour was printed on test region 3. The print layer 4 containing no taste stimulant was printed on the second test region 3' to create a blank sample.

The tester 1 further included an optical identification member 5, which was used to identify the tester by the evaluation software module 9. This optical identification member 5 was arranged on the carrier substrate 2 of the tester 1 and was identifiable by the camera/camcorder 12. In another example embodiment not shown, the optical identification member 5 was visually identifiable, wherein it was formed by a colored array. The optical identification member 5 was further provided with the handling symbol 16, which indicated the direction of handling the tester 1. In another example embodiment not shown, the optical identification member 5 was not provided with the handling symbol 16. The tester 1 prepared in this way was covered with the protective layer 6 in the form of a removable overlay film which overlays the test regions 3, 3'. In another example embodiment not shown, the protective layer 6 was made of a water-soluble film overlaying the test regions 3, 3'. In another example embodiment not shown, the tester 1 was not provided with the protective layer 6.

The testers 1 prepared in this way were used in the test kit 7, which contained 5 testers 1 to test each tastes from the group of: sweet, salty, bitter, sour, and umami.

The test subject is first familiarized with the general instructions on the use of the test kit 7 containing 5 testers 1. In another example embodiment not shown, the test kit 7 comprises two, three, four or six testers 1. The test subject further enters his/her identification data into the evaluation software module 9 and gives consent to the processing of the data entered. Initially, the tester 1. is positioned in the field of view of the camera/camcorder 12 linked to the evaluation device 8. In another example embodiment not shown, the tester 1 is positioned in the field of view of the camera/camcorder 12, which is provided with or connected to the evaluation device 8. The evaluation device 8 is a computer tablet equipped with an integrated touchscreen and front camera/camcorder 12. In another example embodiment not shown, the evaluation device 8 is a mobile phone, laptop, desktop computer, or similar electronic device capable of collecting data from the test subject. The evaluation software module 9 is activated in the evaluation device 8, wherein this module is wirelessly connected to a database 14 containing information about the tester 1. By taking a picture/image of the optical identification member 5 of the tester 1 using the camera/camcorder 12, the evaluation software module 9 identifies the type of tester 1 by comparison with the data in the database 14. Only then, the tester 1 is applied on the test subject's tongue surface by the side with the test region 3, 3', and the taste stimulant contained in the material of the print layer 4 of the test region 3, 3' is tasted. The test subject takes a picture/image using the camera/camcorder 12 with the tester 1 applied on the tongue to evaluate the correct placement of the tester 1. In another example not shown, this step does not occur. The test subject records the result about the taste detected by the taste receptor cells on the tongue surface, what taste do they feel: salty, sweet, bitter sour, or umami. In the evaluation software module 9, in addition, the test subject records the result of differentiation where they sense the taste: on the left, right or both sides of the tongue. In another example embodiment not shown, only taste detection as a positive or negative response to the taste stimulants contained in the print layer 1 of the test region 3,3' is recorded in the evaluation software module 9. In further example embodiment not shown, only taste detection as a positive or negative response to the taste stimulants contained in the print layer 4 of the test region 3,3' is recorded in the evaluation software module 9, wherein the tongue side, where the test subject senses the taste, is also recorded. In further example embodiment not shown, the taste detection by taste receptor cells on the tongue surface is recorded in the evaluation software module 9 as to whether the test subject senses a salty, sweet, bitter, sour or umami taste. The test subject is then prompted by the evaluation software module 9 to rinse their mouth. The recorded result is subsequently sent from the evaluation software module 9 to the external storage device 10 or to the remote server 13 or to the Internet 15 via the communication block 11 after entering the last answer or terminating the use prematurely. In the case of correct use of the test subject's system, the physician is able to determine the result of the test subject's taste sensory evaluation based on the available data. In another example embodiment not shown, data from the evaluation software module 9 is not sent to the external storage device 10.

The external storage 10 is a cloud storage implemented by a database server that communicates with the tablet using the communication block 11 via the HTTPS. In another example embodiment not shown, the HTTP, SSL, or TLS is used. The interface for communication with the physician is implemented by a personal computer that communicates with the database server via HTTPS. In another example embodiment not shown, a mobile phone, computer tablet, or similar electronic device capable of reading data from external storage 10 is used.

Further, the evaluation software module 9 is provided with simple pictorial and textual instructions and guidance for the test subject. The pictorial and textual instruction means, in particular, the instruction to the test subject to place the numerically labeled tester 1 with the taste stimulant applied to the test regions 3, 3' on the tongue, the instruction to select the taste that the test subject senses, whether they sense salty, sweet, bitter, sour, or umami taste, and the lateral distinction of where the test subject senses the taste, whether on the right, left, or both sides. In other example embodiments not shown, the evaluation software module 9 may be provided with the option to instruct the subject to rinse their mouth with water, to read the terms of use of the system, to consent to the processing of personal data, and the like. Receiving responses from the test subject is accomplished by a graphical user interface containing buttons to select the taste stimulant position on the substrate on the right, left or both sides and buttons to select salty, sweet, bitter sour, or umami taste. The control mechanism is implemented by processing the picture/image obtained by the front camera/camcorder 12 of the tablet, wherein the correct position of the tester 1 on the test subject's tongue is checked just during the input of the test subject's responses. The interface for communication with the test subject further sends all relevant data about the ongoing use, including but not limited to test subject identification data, time of use, recorded responses to each taste stimulant substrate including lateral distinction, and information about correctness of execution to the external storage device 10.

The interface for communication with the physician is used to determine the result of the taste sensory self-examination of the test subject and is tailored to display and further process the data available on external storage device 10 by registered physicians. In particular, the interface allows the display of complete data on the test kit 7 use by all test subjects. This data can be filtered by the identification data of the tested subjects, by the time of the tests performed, and so on. The interface also allows for the determination of statistical data needed to determine the result, such as overall accuracy defined as a ratio of correct test subject's responses including lateral distinction to responses from all test subjects, taste accuracy defined as a ration of correct test subject's responses regardless of lateral distinction to responses from all test subjects, and so on.

### Example 2

The taste test kit 7 containing tester 1. to detect the sweet taste was prepared. The tester 1 for sweet taste detection was prepared as follows. The print layer 4 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 1 of the test region 3 was prepared, which further contained 10% w/w of sucrose as the sweet taste stimulant. In another example embodiment not shown, glucose, fructose, sorbitol, aspartame, sodium cyclamate, saccharin, acesulfame potassium, sucralose, thaumatin, neotame, lugduname, and/or combinations thereof were used as the taste stimulant to detect the sweet taste. This allowed 12.5 mg of sucrose to be printed on tester 1 per test region 3 of the taste stimulant.

Polyethylene film or PET film of a thickness of 150 µm and a width of 150 mm served as the carrier substrate 2 of tester 1 was stretched on a screen printing machine allowing roll-to-roll or R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 of the sweet taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 3

The taste test kit 7 containing tester 1 to detect the bitter taste was prepared. The tester 1 for bitter taste detection was prepared as follows. The print layer 4 of test region 3' was prepared and contained a water-soluble cellulose derivative-based polymer containing 9% w/w of cellulose derivative, 10% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 0.6% w/w of quinine as the bitter taste stimulant. In another example embodiment not shown, 6-n-propylthiouracil, phenylthiourea, sucrose octacetate, caffeine, strychnine, naringin, neohesperidine, humulone, and/or combinations thereof were used as the taste stimulant to detect the bitter taste. This allowed 0.3 mg of quinine to be printed on tester 1 per test region 3 of the taste stimulant.

Polyethylene naphthalate film or PEN film of a thickness of 125 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polycarbonate, polypropylene, polyethylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a red graphic motif facilitating image analysis as well as manipulation for the test subject. The print motif was printed using a UV-curable printing formulation using a 275-mesh screen. As a second layer, a layer of test region 3 of the bitter taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 4

The taste test kit 7 containing tester 1. to detect the sour taste was prepared. The tester 1. for sour taste detection was prepared as follows. The print layer 1 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 6% w/w of citric acid as the bitter taste stimulant. In another example embodiment not shown, acetic acid, ethylenediaminetetraacetic acid, tartaric acid, phosphoric acid, ascorbic acid, and/or combinations thereof were used as the taste stimulant to detect the sour taste. This allowed 11.5 mg of citric acid to be printed on tester 1 per test region 3 of the taste stimulant.

PET film of a thickness of 150 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 of the sour taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 5

The taste test kit 7 containing tester 1 to detect the salty taste was prepared. The tester 1 for salty taste detection was prepared as follows. The print layer 4 of test region 3' was prepared and contained a water-soluble cellulose derivative-based polymer containing 9% w/w of cellulose derivative, 10% w/w of glycol, based humectant, 10% w/w of monofunctional alcohol. This formulation was used to print the blank layers, i.e. a part of the test region 3' of tester 1 without the taste stimulants. Subsequently, a similar print layer 1 of the test region 3 was prepared, which further contained 8% w/w of sodium chloride as the salty taste stimulant. In another example embodiment not shown, KCI, KBr, NaBr, LiCI, LiBr, Lil, NaNO₃, KNO₃, L-ornithyl-β-alanine, and/or combinations thereof were used as the taste stimulant to detect salty taste. This allowed 12.5 mg of sodium chloride to be printed on tester 1 per test region 3 of the taste stimulant.

PEN film of a thickness of 125 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polycarbonate, polypropylene, polyethylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a red graphic motif facilitating image analysis as well as manipulation for the test subject. The print motif was printed using a UV-curable printing formulation using a 275-mesh screen. As a second layer, a layer of test region 3 the salty taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 6

The taste test kit 7 containing tester 1 to detect the salty taste was prepared. The tester 1 for salty taste detection was prepared as follows. The print layer 4 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 6% w/w of sodium chloride as the salty taste stimulant. In another example embodiment not shown, KCI, KBr, NaBr, LiCI, LiBr, Lil, NaNO₃, KNO₃, L-ornithyl-β-alanine, and/or combinations thereof were used as the taste stimulant to detect salty taste. This allowed 10 mg of sodium chloride to be printed on tester 1 per test region 3 of the taste stimulant.

PET film of a thickness of 150 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 the salty taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 7

The taste test kit 7 containing tester 1 to detect the bitter taste was prepared. The tester 1 for bitter taste detection was prepared as follows. The print layer 4 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 0.1% w/w of humulone as the bitter taste stimulant. In another example embodiment not shown, 6-n-propylthiouracil, phenylthiourea, sucrose octacetate, caffeine, strychnine, naringin, neohesperidine, quinine, and/or combinations thereof were used as the taste stimulant to detect the bitter taste. This allowed 0.1 mg of humulone to be printed on tester 1 per test region 3 of the taste stimulant.

PET film of a thickness of 150 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 of the bitter taste stimulant, from the printing formulation mentioned above, was printed using coarser screen printing. Printing a third layer of test region 3' followed, wherein this layer did not contain the taste stimulant of the formulation composition of which is described above. When printing the second and third layers, drying was always carried out in a drying zone of a temperature of 110 °C.

### Example 8

The taste test kit 7 containing tester 1 to detect the salty taste was prepared. The tester 1 for salty taste detection was prepared as follows. The print layer 4 of test region 3' was prepared and contained a water-soluble pectin polymer containing 5% w/w of pectin, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 6% w/w of sodium chloride as the salty taste stimulant. In another example embodiment not shown, KCl, KBr, NaBr, LiCl, LiBr, Lil, NaNO₃, KNO₃, L-ornithyl-β-alanine, and/or combinations thereof were used as the taste stimulant to detect salty taste. This allowed 12.5 mg of sodium chloride to be printed on tester 1 per test region 3 of the taste stimulant.

PET film of a thickness of 150 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 the salty taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 9

The taste test kit 7 containing tester 1 to detect the bitter taste was prepared. The tester 1. for bitter taste detection was prepared as follows. The print layer 4 of test region 3' was prepared and contained a water-soluble polyvinyl pyrrolidone copolymer containing 11% w/w of polyvinyl pyrrolidone, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar print layer 4 of the test region 3 was prepared, which further contained 0.3% w/w of quinine as the bitter taste stimulant. In another example embodiment not shown, 6-n-propylthiouracil, phenylthiourea, sucrose octacetate, caffeine, strychnine, naringin, neohesperidine, humulone, and/or combinations thereof were used as the taste stimulant to detect the bitter taste. This allowed 0.25 mg of quinine to be printed on tester 1 per test region 3 of the taste stimulant.

PET film of a thickness of 150 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polyethylene naphthalate, polycarbonate, polypropylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a blue graphic motif facilitating image analysis as well as manipulation for the test subject. The print motif was printed using a UV-curable printing formulation using a 275-mesh screen. As a second layer, a layer of test region 3 of the bitter taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C.

### Example 10

The taste test kit 7 containing tester 1 to detect the salty taste was prepared. The tester 1 for salty taste detection was prepared as follows. The print layer 4 of test region 3' was prepared and contained a water-soluble cellulose derivative-based polymer containing 9% w/w of cellulose derivative, 10% w/w of glycol-based humectant, 10% w/w of monofunctional alcohol. This formulation was used to print the blank layers, i.e. a part of the test region 3' of tester 1 without the taste stimulants. Subsequently, a similar layer 4 of the test region 3 was prepared, which further contained 8% w/w of sodium chloride as the salty taste stimulant. In another example embodiment not shown, KCI, KBr, NaBr, LiCI, LiBr, Lil, NaNO₃, KNO₃, L-ornithyl-β-alanine, and/or combinations thereof were used as the taste stimulant to detect salty taste. This allowed 12.5 mg of sodium chloride to be printed on tester 1 per test region 3 of the taste stimulant.

PEN film of a thickness of 125 µm and a width of 150 mm served as the carrier substrate 2 of tester 1 was stretched on a screen printing machine allowing R2R printing. In other example embodiment not shown, the carrier substrate 2 was made of a polymeric film selected from the group of: polyethylene terephthalate, polyvinyl chloride, polycarbonate, polypropylene, polyethylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. A series of motifs from three screen printing units were printed on substrate 2 in the register. The first print layer represented the optical identification member 5 in the form of a red graphic motif facilitating image analysis as well as manipulation for the subject being tested. The print motif was printed using a UV-curable printing formulation using a 275-mesh screen. As a second layer, a layer of test region 3 the salty taste stimulant, from the printing formulation mentioned above, was printed using coarser screen printing. Printing a third layer of test region 3' followed, wherein this layer did not contain the taste stimulant of the formulation, composition of which is described above. When printing the second and third layers, drying was always carried out in a drying zone of a temperature of 110 °C. The tester 1 of the test kit 7 prepared in this way was then covered with the protective layer 6 in the form of a 30 µm thin polyethylene film or PE film.

### Example 11

According to Example 2, 4 testers 1 forming the test kit 7 were successively prepared by printing 4% w/w of glucose on the test region 3, 3' as the sweet taste stimulant to detect the sweet taste, printing 6% w/w sodium chloride on the test region 3, 3' as the salty taste stimulant to detect the salty taste, printing 0.5% w/w quinine on the test region 3, 3' as the bitter taste stimulant to detect the bitter taste, and printing 4% w/w citric acid on the test region 3, 3' as the sour taste stimulant to detect the sour taste. In the final step, the testers 1 of the test kit 7 prepared in this way were covered with the protective layer 6 in the form of a polypropylene film or PP film of a thickness of 40 µm.

### Example 12

The taste test kit 7 containing tester 1. to detect the sweet taste was prepared. The tester 1 for sweet taste detection was prepared as follows. The print layer 4 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar formulation was prepared, which further contained 10% w/w of sucrose as the sweet taste stimulant, forming the test region 3. In another example embodiment not shown, glucose, fructose, sorbitol, aspartame, sodium cyclamate, saccharin, acesulfame potassium, sucralose, thaumatin, neotame, lugduname, and/or combinations thereof were used as the taste stimulant to detect the sweet taste. This allowed 12.5 mg of sucrose to be printed on tester 1 per test region 3 of the taste stimulant. The sweet taste detection formulation prepared in this way was printed on the carrier substrate 2 using microdispensing technique.

### Example 13

The taste test kit 7 containing tester 1 to detect the sweet taste was prepared. The tester 1 for sweet taste detection was prepared as follows. The print layer 4 of test region 3' was prepared and contained a water-soluble polysaccharide-based polymer containing 8% w/w of polysaccharide, 0.2% w/w of volatile surfactant, 15% w/w of glycol-based humectant, 10% w/w of monofunctional alcohol. This formulation was used to print the blank layers, i.e. a part of the test region 3' of tester 1 without the taste stimulants. Subsequently, a similar formulation was prepared, which further contained 10% w/w of sucrose as the sweet taste stimulant, forming the test region 3. In another example embodiment not shown, glucose, fructose, sorbitol, aspartame, sodium cyclamate, saccharin, acesulfame potassium, sucralose, thaumatin, neotame, lugduname, and/or combinations thereof were used as the taste stimulant to detect the sweet taste. This allowed 2.5 mg of sucrose to be printed on tester 1 per test region 3 of the taste stimulant. The sweet taste detection formulation prepared in this way was printed on the carrier substrate 2 using flexographic printing technique with the printing formulation, viscosity of which was reduced by the addition of 30% w/w of water.

### Example 14

The taste test kit 7 containing testers 1 to detect the bitter taste was prepared. A plastic spoon was used as the carrier substrate 2 of the given testers 1, wherein all layers, namely the test region 3, 3', the print layer 4, the optical identification member 5, and the protective layer 6, were printed on the spoon curved 3D surface. The tester 1 for bitter taste detection was prepared as follows. A printing formulation was prepared and contained a water-soluble cellulose derivative-based polymer containing 8% w/w of cellulose derivative, 10% w/w of glycol, based humectant, 10% w/w of monofunctional alcohol. This formulation was used to print the blank layers, i.e. a part of the test region 3' of tester 1 without the taste stimulants. Further, the test region 3 having a similar formulation which further contained 0.6% of quinine as the bitter taste stimulant was prepared. In another example embodiment not shown, 6-n-propylthiouracil, phenylthiourea, sucrose octacetate, caffeine, strychnine, naringin, neohesperidine, humulone, and/or combinations thereof were used as the taste stimulant to detect the bitter taste. This allowed 0.3 mg of quinine to be printed on tester 1 per test region 3 of the taste stimulant. The given formulations were prepared for printing by microdispensing technique.

A spoon made of polypropylene material was printed on a pad printing machine allowing printing on 3D surfaces; this spoon served as the substrate 2 of tester 1. In other example embodiment not shown, the carrier substrate 2 was made of a polymeric film selected from the group of: polyethylene terephthalate, polyvinyl chloride, polyethylene naphthalate, polycarbonate, polyethylene, polyetheretherketone, polystyrene, and/or combinations thereof. In another example embodiment not shown, the carrier substrate 2 was formed by a biocompatible polymer selected from the group of: polysaccharide, proteins, cellulose and/or derivatives thereof, pectins, and/or combinations thereof. The first print layer represented the optical identification member 5 in the form of a blue graphic motif facilitating image analysis as well as manipulation for the test subject. The print motif was printed using a UV-curable printing formulation. As a second layer, a layer of test region 3 of the bitter taste stimulant, from the printing formulation mentioned above, was printed using microdispensing technique. Printing a third layer of test region 3' followed, wherein this layer did not contain the taste stimulant of the formulation composition of which is described above; the microdispensing technique was used as well. When printing the second and third layers, drying was always carried out in a drying tunnel of a temperature of 110 °C.

### Example 15

The procedure as in Example 14 was repeated, wherein the test regions 3, 3' for the blank and for the bitter taste detection stimulant were printed using a pad printing technique.

### Industrial Applicability

A taste tester, a test kit comprising the tester, a method for evaluating data collection from an application of the test kit, and a system for collecting the data according to the present invention can be used for self-examination of individual test subjects or examination by a physician to evaluate the activity of taste receptor cells on the tongue surface.

### Reference Signs List

- 1: Tester
- 2: Carrier substrate
- 3: Test region
- 3': Test region
- 4: Print layer
- 5: Optical identification member
- 6: Protective layer
- 7: Test kit
- 8: Evaluation device
- 9: Evaluation software module
- 10: External storage device
- 11: Communication block
- 12: Camera/camcorder
- 13: Remote server
- 14: Database
- 15: Internet
- 16: Handling symbol

## Claims

1. The tester (1) for taste testing **characterized in that** it comprises the carrier substrate (2), on which at least one test region (3, 3') is formed by the print layer (4) made of a material comprising a taste stimulant.

2. The tester (1) according to claim 1 **characterized in that** the print layer (4) material comprises taste stimulant for detecting sweet taste selected from the group of: sucrose, glucose, fructose, sorbitol, aspartame, sodium cyclamate, saccharin, potassium acesulfame, sucralose, thaumatin, neotame, lugduname, and/or combinations thereof.

3. The tester (1) according to claim 1 **characterized in that** the print layer (4) material comprises taste stimulant for detecting the salty taste selected from the group of: NaCl, KCI, KBr, NaBr, LiCI, LiBr, Lil, NaNO₃, KNO₃, L-ornithyl-β-alanine, and/or combinations thereof.

4. The tester (1) according to claim 1 **characterized in that** the print layer (4) material comprises taste stimulant for detecting bitter taste selected from the group of: quinine, 6-n-propylthiouracil, phenylthiourea, sucrose octaacetate, caffeine, strychnine, naringin, neohesperidine, humulone, and/or combinations thereof.

5. The tester (1) according to claim 1 **characterized in that** the print layer (4) material comprises taste stimulant for detecting sour taste selected from the group of: citric acid, acetic acid, ethylenediaminetetraacetic acid, tartaric acid, phosphoric acid, ascorbic acid, and/or combinations thereof.

6. The tester (1) according to claim 1 **characterized in that** the print layer (4) material comprises taste stimulant for detecting the umami taste selected from the group of: monosodium glutamate, potassium glutamate, and/or combinations thereof.

7. Tester (1) according to any of claims 1 to 6 **characterized in that** there are two test regions (3, 3') arranged in parallel to each other at a distance of 1 to 30 mm on the carrier substrate (2) to examine the taste bilaterally.

8. The tester (1) according to claim 7 **characterized in that** there are two test regions (3, 3') on the carrier substrate (2), wherein the first test region (3) and the second test region (3) are formed by the print layer (4) made of a material comprising the same taste stimulants.

9. The tester (1) according to claim 7 **characterized in that** there are two test regions (3, 3') on the carrier substrate (2), wherein the first test region (3) is formed by the print layer (4) made of a material comprising a taste stimulant, and the second test region (3`) is formed by the print layer (4) made of a material comprising a taste stimulant different from the taste stimulant of the first test region (3).

10. The tester (1) according to claim 7 **characterized in that** there are two test regions (3, 3') on the carrier substrate (2), wherein the first test region (3) is formed by the print layer (4) made of a material comprising a taste stimulant, and the second test region (3`) is formed by the print layer made of a material not comprising the taste stimulant.

11. The tester (1) according to any of claims 1 to 10, further **characterized in that** it further comprises optical identification member (5) arranged on the carrier substrate (2), wherein this member is identifiable visually or by a camera/camcorder (12).

12. The tester (1) according to claim 11 **characterized in that** the optical identification member (5) is a colored array.

13. The tester (1) according to claim 11 or 12 **characterized in that** the optical identification member (5) is provided with a handling symbol (16) indicating the tester (1) handling direction.

14. The tester (1) according to any of claims 1 to 13 **characterized in that** it comprises further the protective layer (6) in the form of an overlaying water-soluble film arranged on the carrier substrate (2) and overlaying the test region (3, 3').

15. The tester (1) according to any of claims 1 to 13 **characterized in that** it comprises further the protective layer (6) in the form of a removable overlay film arranged on the carrier substrate (2) and overlaying the test region (3, 3').

16. The tester (1) according to any of claims 1 to 15 **characterized in that** the carrier substrate (2) is formed by a polymeric film selected from the group of: polyethylene terephthalate, polyvinyl chloride, polyethylene naphthalate, polycarbonate, polypropylene, polyethylene, polyetheretherketone, polystyrene, and/or combinations thereof.

17. The tester (1) according to any of claims 1 to 15 **characterized in that** the carrier substrate (2) is formed by a biocompatible polymer selected from the group of: polysaccharide, proteins, cellulose and/or derivatives thereof, pectins, and/or combinations thereof.

18. The test kit (7) comprising at least two testers (1) for taste testing **characterized in that** it comprises at least two testers (1) according to any of claims 1 to 17.

19. The test kit (7) according to claim 17 **characterized in that** it comprises 5 testers (1), each comprising the test region (3, 3') formed by the print layer (4) made of a material comprising a different taste stimulant.

20. The test set (7) according to claim 19 **characterized in that** one tester (1) comprises the test region (3, 3') formed by the print layer (4) provided with the taste stimulant for detecting sweet taste, the second tester (1) comprises the test region (3, 3') formed by the print layer (4) provided with the taste stimulant for detecting salty taste, the third tester (1) comprises the test region (3, 3') formed by the print layer (4) provided with the taste stimulant for detecting bitter taste, the fourth tester (1) comprises the test region (3, 3') formed by the print layer (4) provided with the taste stimulant for detecting sour taste, and the fifth tester (1) comprises the test region (3, 3') formed by the print layer (4) provided with the taste stimulant for detecting umami taste.

21. The method of data collection from the tester (1) according to any of claims 11 to 17 and/or the test kit (7) according to any of claims 18 to 20 **characterized in that** it comprises successive procedure steps:
a) The tester (1) is placed into the field of view of a camera/camcorder (12) equipped with or associated with the evaluation device (8), which includes the evaluation software module (9) linked to the database (14) containing tester (1) information and the communication block (11) for remote communication;
b) The camera/camcorder (12) is activated and a picture/image of the optical identification member (5) of the tester (1) is taken;
c) The picture/image is transferred to the evaluation software module (9), which identifies the tester (1) type by comparing it with the data in the database (14);
d) With the test region (3, 3'), the tester (1) is placed on the tongue surface of the subject being tested;
e) The test subject tastes the taste stimulants contained in the print layer (4) material forming the test region (3, 3');
f) The result of the tasting is recorded in the evaluation software module (9) as information about a positive or negative reaction to the taste stimulants contained in the print layer (4) of the test region (3, 3');
g) The data from the evaluation software module (9) is sent from the communication block (11) to an external storage device (10) or the remote server (13) or to the Internet (15).

22. The method according to claim 21 **characterized in that** the camera/camcorder (12) is activated between procedure steps d) and e) and a picture/image of the test subject with the tester (1) applied on the tongue is taken to evaluate the correct tester (1) placement.

23. The method according to claim 21 or 22 **characterized in that** the taste type perceived during the tasting is also recorded as the result of the tasting in procedure step f).

24. The method according to any of claims 21 to 23 **characterized in that** the left or right side of the tongue is also recorded as the result of the tasting in procedure step f).

25. The method according to claim 24 **characterized in that** a particular taste type perceived by the left or right side of the tongue is also recorded as the result of the tasting in procedure step f).

26. The system for performing the method of data collection according to any of claims 21 to 25 **characterized in that** it comprises at least one tester (1) according to any of claims 1 to 17 or at least one test kit (7) according to any of claims 18 to 20, further comprising the camera/camcorder (12) to take picture/image of the optical identification member (5) of the tester (1), further comprising the evaluation device (8) connected to the camera/camcorder (12), wherein the evaluation device (8) comprises the evaluation software module (9) to evaluate the picture/image of the optical identification member (5) of the tester (1), linked to the database (14) containing stored information on the testers (1), and the communication block (11) linked to the evaluation software module (9) to send data from the evaluation software module (9).

27. The system according to claim 26 **characterized in that** it comprises further the external storage device (10) to store data sent via the communication block (11) from the evaluation software module (9).

28. The system according to claim 26 **characterized in that** it comprises further the remote server (13) to store data sent via the communication block (11) from the evaluation software module (9).

29. The system according to claim 26 **characterized in that** it comprises further the means for connecting and transmitting data to the Internet (15) sent via the communication block (11) from the evaluation software module (9).
